# EUROPÄISCHE PATENTSCHRIFT

(11) **EP 0 184 684 B1**
(45) Veröffentlichungstag und Bekanntmachung des Hinweises auf die Patenterteilung: **21.04.1993**
(21) Anmeldenummer: 85114462.6
(22) Anmeldetag: 14.11.1985
(51) Int. Cl.: A61M 25/02

(54) **Mandrin zum Versteifen von Sonden**
Mandrel to stiffen probes
Mandrin pour rigidifier des sondes

(30) Priorität: 08.12.1984 DE 3444935
(43) Veröffentlichungstag der Anmeldung: 18.06.1986
(73) Patentinhaber: N.V. Nutricia, NL-2712 HM Zoetermeer (NL)
(72) Erfinder: Iwatschenko, Peter, D-8524 Neunkirchen (DE)
(74) Vertreter: von Hellfeld, Axel, Dr. Dipl.-Phys.

(56) Entgegenhaltungen:
- FR-A- 2 399 879
- GB-A- 1 067 257
- US-A- 3 731 671

## Beschreibung

Die Erfindung betrifft eine Sonde aus leicht biegbaren Werkstoffen, mit einem Mandrin in Form eines Drahtes zum Versteifen der Sonde bei deren Einführung in Körperhöhlen, wobei der Mandrin nach dem Anlegen der Sonde wieder entfernt wird.

Aus der FR-A-2 399 879 ist eine Sonde bekannt, bei der die äußere Oberfläche der Sonde selbst (also nicht die Oberfläche eines Mandrins) mit einem Überzug aus einem physiologisch verträglichen Stoff versehen ist, der die Gleiteigenschaften der Sonde verbessert, so daß diese einfacher in eine Körperhöhlung einzuführen ist.

Aus der DE-A-2 851 547 ist es bekannt, einer Gelatine einen Anteil an physiologisch verträglichem Alkohol als Weichmacher beizufügen oder der Gelatine einen Anteil an Formaldehyd oder einem anderen Aldehyd beizufügen, um ein zu frühes Auflösen der Gelatine zu vermeiden.

Die hier in Rede stehenden, leicht biegbaren und hoch flexiblen Sonden bestehen im allgemeinen aus einem hochmolekularen Werkstoff, wie Silikonkautschuk oder Polyurethan, Latex oder dgl., der die notwendige physiologische Unbedenklichkeit aufweist und auch über einen relativ geringen Radius biegbar ist, ohne hierbei einzuknicken. Der Begriff "Sonde" ist hierbei im weitesten Sinne des Wortes zu werten, und er umfaßt auch Katheter und Endoskope.

An solche Sonden werden zwei unterschiedliche, miteinander konkurrierende Anforderungen gestellt. Zum einen müssen sie die notwendige Steifigkeit aufweisen, um einwandfrei, d.h. in einer den Patienten weitgehend schonenden Weise in dessen Körperhöhlung eingeführt werden zu können. Dem kommt insbesondere dann eine besondere Bedeutung zu, wenn der Patient das Einführen nicht durch Schluckbewegungen unterstützen kann, bzw. wenn das Einführen nur unter Oberwindung von Körperreflexen möglich ist. Andererseits muß eine solche Sonde im eingeführten Zustand weich und flexibel sein, da sie unter Umständen über längere Zeiträume im Körper des Patienten verbleibt und diesen weder stören, noch in seiner Bewegungsfreiheit behindern darf. Verbleiben solche Sonden mehrere Tage im Magen oder Duodenum oder auch in anderen Körperhöhlen, so dürfen sie auch nicht durch den Verlust von ihre Weichheit bewirkenden Mitteln hart werden, da andernfalls Drucknekrosen auftreten können. Eine Sonde muß hinreichend auch um einen geringen Radius, beispielsweise von 20 mm einwandfrei selbst um einen Winkel von mehr als 90° biegbar sein, um sich den Biegungen der Körperhöhlen, insbesondere bei einem Zugangsweg durch den Nasen- Rachenraum, anpassen zu können.

Zum Zwecke ihrer Versteifung beim Einführen in Körperhöhlen werden beispielsweise PVC-Sonden vor dem Einführen gekühlt (DE-A-21 40 994). Alternativ hierzu steht die Versteifung der Sonden mittels eines Drahtes, der nach dem Einführen der Sonde aus dieser wieder entfernt wird. Es ist hierbei von Nachteil, daß das Einbringen des Drahtes in die Sonde aufwendige Handhabungen erfordert und die Gefahr des Durchstoßens der dünnen Sondenwandung durch den einen nur geringen Querschnitt aufweisenden Draht besteht. Ein durch die Sondenwandung dringendes Drahtende kann zu unangenehmen Verletzungen des Patienten führen. Es ist von besonderer Bedeutung, daß zwischen Draht und Sonde zunächst eine möglichst hohe Reibung besteht, um Relativbewegungen zwischen Draht und Sonde beim Einführvorgang weitgehend auszuschließen. Gerade dieser Gesichtspunkt aber steht im diametralen Gegensatz zu der Forderung, den REibungskoeffizienten zwischen Sonde und Draht möglichst gering zu halten, um den Draht nach dem Anlegen der Sonde möglichst mühelos wieder entfernen zu können.

In BSI 63 14/1983 werden beispielsweise maximale Kräfte von 5 N zum Entfernen eines solchen in einer Sonde angeordneten Versteifungsdrahtes vorgeschrieben. Eine Lösung dieses Problems zeigt US-PS 397 535 auf. Eine andere Lösung, die die Anwendung eines Drahtes entbehrlich macht, ist in DE-PS 28 51 547 beschrieben. Gleichwohl gibt es Fälle, die die Anwendung einer dort offenbarten Sonde verbieten, wie es andererseits in bestimmten Fällen unerläßlich ist, mit einem Mandrin versteifte Sonden zu verwenden.

Die der Erfindung zugrunde liegende Aufgabe befaßt sich im Rahmen dieses letztgenannten Falles mir der Ausbildung eines Mandrins der eingangs bezeichneten Art mit dem Ziel, die Handhabung einer drahtversteiften Sonde in solcher WEise zu verbessern, daß das Einführen gefahrloser und schonender vonstatten geht und andererseits der Mandrin wesentlich leichtgängiger als bisher aus der Sonde wieder entfernbar ist. Hierdurch wird die Gefahr von Verletzungen des Patienten auf ein Mindestmaß reduziert und eine besonders schonende Behandlung gewährleistet. Zur Lösung dieses Problems sieht die Erfindung einen Mandrin der oben bezeichneten Art vor, bei welchem der Draht einen Oberzug aus einem physiologisch verträglichen Stoff aufweist, von dem zumindest die Oberfläche hydrophil ist. Ein solcher Draht ist verhältnismäßig dünn. Er weist im allgemeinen einen Durchmesser von 0,2 - 0,4 mm auf, und der Überzug kann, indem er durch ein Tauchverfahren oder durch Aufsprühen auf den Draht aufgebracht wird, so dünn gehalten werden, daß eine ausreichende Leichtgängigkeit des Mandrins bei seinem Einführen in die Sonde gewährleistet ist. Die Hydrophilie, zumindest der Oberfläche des Überzuges, wenn nicht des gesamten Überzugwerkstoffes, ist erforderlich, um die vergleichsweise hohe Reibung zwischen dem erfindungsgemäßen Mandrin und der Innenwandung der Sonde dadurch wirksam herabzusetzen, daß unmittelbar vor der Entnahme des Mandrins aus der angelegten Sonde in diese eine physiologisch verträgliche Flüssigkeit, wie die verschiedenen Körpersäfte oder Wasser eingebracht werden. Durch die hydrophile Eigenschaft der Drahtoberfläche läßt sich nun der Mandrin bereits bei Anwendung vergleichsweise recht geringer Zugkräfte aus der angelegten Sonde ohne weiteres entfernen. Versuche haben ergeben, daß bei Anwendung eines in der erfindungsgemäßen Weise ausgebildeten Mandrins dieser im Test nach BSI mit einer Kraft von 0,5 N bis maximal 1 N entfernt werden kann. Hierdurch wird eine wesentliche Verbesserung gegenüber gebräuchlichen drahtversteiften Sonden erreicht, die ebenso dem Patienten wie dem Behandlungspersonal zugute kommt und das Risiko des Anlegens einer solchen Sonde bedürfender Behandlungen entscheidend zu mindern in der Lage ist.

Es liegt im Rahmen der Erfindung, Gelatine als Überzug für den den Mandrin bildenden Draht zu verwenden. Gelatine ist ein billig zur Verfügung stehendes, problemlos zu verarbeitendes Material, das darüber hinaus geschmacksneutral ist, so daß es praktisch überall angewendet werden kann. Es liegt im Rahmen dieses Erfindungsgedankens, der den Überzug des Drahtes bildenden Gelatine einen Anteil an physiologisch verträglichem Alkohol als Weichmacher zuzusetzen. Hierzu kommt beispielsweise Sorbit ebenso in Betracht, wie ein mehrwertiger Alkohol, beispielsweise Glyzerin. Im allgemeinen ist ein Zusatz von 0,5 - 5 % Glyzerin ausreichend. Gegebenenfalls kann der Gelatine ein Anteil an Formaldehyd oder eines anderen Aldehyds zugesetzt werden, um die Löslichkeit der Gelatine herabzusetzen.

In weiterer Ausgestaltung des erfindungsgemäßen Mandrins kann an dem äußeren Ende dessen Drahtes ein Konnektor befestigt sein, der je eine Buchse zum Befestigen des Sondenendes und zum Einführen eines Gleitmittels, sowie einen beide Buchsen miteinanderverbindenden Kanal aufweist. Ein in dieser Weise ausgebildeter Mandrin ermöglicht ein besonders müheloses Einführen der als Gleitmittel zwischen Sonde und Mandrin dienenden Flüssigkeit sowie darüber hinaus das Aufbringen der zur Entnahme des Mandrins erforderlichen Zugkraft.

Zweckmäßig weist der Konnektor eine Halterung zur Aufnahme einer an einem Drahtende angebrachten Schlinge auf. Der Konnektor kann dann leicht an dieser Halterung befestigt werden, was das Aufbringen der zur Entnahme des Mandrins erforderlichen Zugkraft wesentlich erleichert.

Es hat sich als besonders vorteilhaft erwiesen, die Anordnung so zu treffen, daß der Konnektor aus zwei etwa in einer Axialebene zusammensetzbaren Teilen besteht, in deren Trennebene eine Ausnehmung zur Aufnahme der Drahtschlinge und des die beiden Buchsen verbindenden Kanals ausgebildet sind. Zum Einlegen der Drahtschlinge werden die beiden Teile auseinandergetrennt und über der Drahtschlinge des Mandrins wieder geschlossen.

Es liegt im Rahmen der Erfindung, daß die eine Buchse des - Konnektors als Paßsitz für den Austrittsstutzen einer Injektionsspritze ausgebildet ist. Dadurch läßt sich das Gleitmittel in besonders zuverlässiger Weise in das Innere der Sonde einführen.

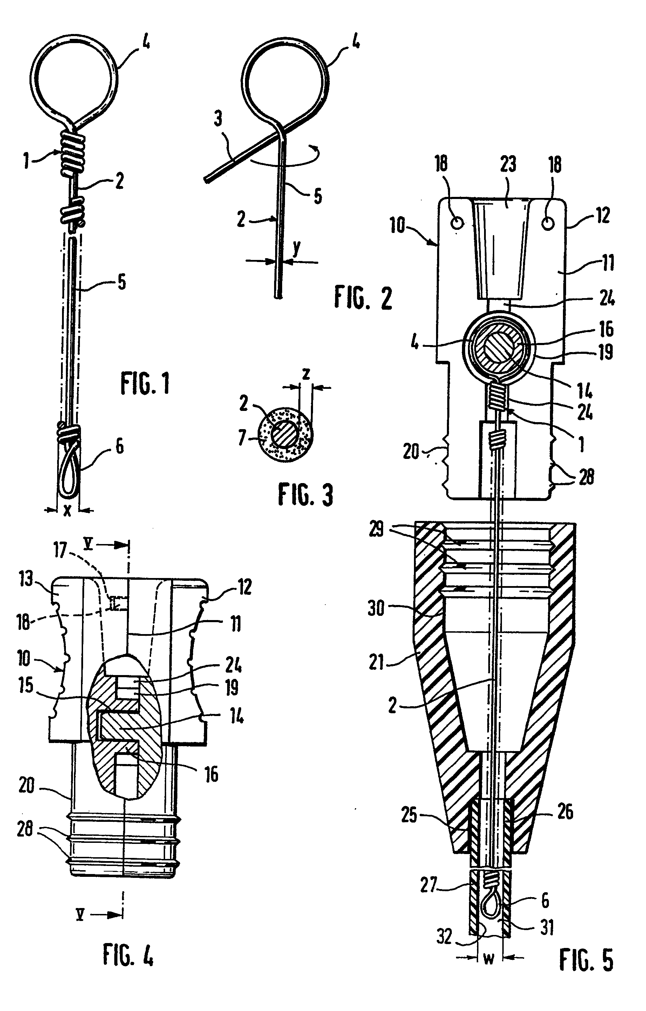

Weitere Vorteile, Merkmale und Einzelheiten der Erfindung ergeben sich aus der folgenden Beschreibung einer bevorzugten Ausführungsform der Erfindung, sowie anhand der Zeichnung. Hierbei zeigen:
- Fig. 1: eine Ausführungsform des erfindungsgemäßen Mandrins;
- Fig. 2: ein Detail des Mandrins;
- Fig. 3: einen Schnitt durch den Draht des Mandrins;
- Fig. 4: eine teilweise aufgebrochene Seitenansicht des Konnektors und
- Fig. 5: einen Schnitt durch den Konnektor nach Linie V-V in Fig. 4
Der Mandrin 1 ist in der Weise aus einem Draht 2 aus rostfreiem und physiologisch unbedenklichem Werkstoff gebildet, daß das eine Drahtende 3 zu einer Schlinge 4 verformt und, wie aus Fig. 1 ersichtlich, um den gestreckten Schaft 5 des Drahtes 2 aufgewickelt wird. Das entgegengesetzte Ende des Mandrins 1 wird gleichfalls zu einer Schlinge 6 verformt, deren maximaler Durchmesser jedoch wesentlich geringer ist als derjenige der Schlinge 4. Das Drahtende wird dann um den gestreckten Schaft 5 in Form einer Wendel aufgewickelt, so daß praktisch der gesamte Schaft 5 des Drahtes 2 bewickelt ist. Der Durchmesser x des Mandrins 1 entspricht dann etwa demjenigen der Schlinge 6 am Einführende. Er mag etwa 0,9 mm betragen.

Der zur Ausbildung des Mandrins 1 dienende Draht 2 hat einen Durchmesser y von etwa 0,05 mm, und er ist mit einem Überzug 7 aus einem physiologisch verträglichem Stoff, beispielsweise Gelatine versehen, dessen Dicke z 0,1 - 0,2 mm beträgt.

Die Drahtschlinge 4 am äußeren Ende des Mandrins 1 wird an dem Konnektor 10 befestigt. Dieser Besteht aus den beiden, in einer Axialebene 11 trennbaren Teilen 12 und 13, von denen das Teil 12 einen Zapfen 14 zum Eingriff eines in die Bohrung 15 einer Buchse 16 des anderen Teils dient. Die hierdurch gebildete Steckverbindung wird durch zwei weitere in entsprechende Bohrungen 17 des Teils 13 eingreifende Steckzapfen 18 vervollständigt. In der Trennebene 11 der beiden Teile 12 und 13 ist eine der Formgebung der Schlinge 4 des Mandrins 1 entsprechende Ausnehmung 19 angeordnet, die die Buchse 16 des Teils 13 umgibt.

An dem Konnektor 10 sind zwei Kopplungselemente vorgesehen, nämlich ein Zapfen 20 zum Anschluß eines Mundstücks 21 am Ende 22 der Sonde 6 und eine Buchse 23, die als Paßsitz für den nicht dargestellten Austrittsstutzen einer Injektionsspritze od. dgl. dient. Beide Kopplungselemente 20 und 23 sind durch einen Kanal 24 miteinander verbunden, der die die Drahtschlinge 4 aufnehmende Ausnehmung 19 mit einschließt. Das Mundstück 21 ist mit einer Buchse 25 versehen, die das Ende 26 der Sonde 27 fest aufnimmt. Das Mundstück 21 erweitert sich kegelförmig etwa auf den Durchmesser des Zapfens 20 und kann im Paßsitz auf diesem befestigt werden, wobei die Rippen 28 am Außenumfang des Zapfens 20 in Eingriff mit den rinnenartigen Vertiefungen 29 an der Innenseite 30 des Mundstücks 21 gelangen.

Durch Ansetzen einer Spritze an der Buchse 23 des Konnektors 10 kann ein flüssiges Gleitmittel in den Innenraum 31 der Sonde 27 eingeleitet werden, dessen Durchmesser w wenig größer ist als der größte Außendurchmesser x des Mandrins 1. Dieses Gleitmittel, welches eine Körperflüssigkeit oder auch Wasser sein kann, vermindert im Hinblick auf die hydrophilen Eigenschaften des Überzugs 7 des Mandrins 1 die Reibung zwischen dem Mandrin 1 und der Innenwand 32 der Sonde 27.

Der Grundgedanke der Erfindung, den zur Ausbildung des Mandrins 1 dienenden Draht 2 mit einem hydrophilen Überzug zu versehen, läßt sich selbstverständlich auch mit einem Mandrin anderer als der dargestellten Gestaltung verwirklichen, und es ist auch der Konnektor in einer anderen Ausgestaltung vorstellbar oder auch ganz entbehrlich.

## Patentansprüche

1. Sonde (6) aus leicht biegbaren Werkstoffen, mit einem Mandrin in Form eines Drahtes (2) zum Versteifen der Sonde bei deren Einführung in Körperhölen, wobei der Mandrin nach dem Anlegen der Sonde wieder entfernt wird,
dadurch **gekennzeichnet,** daß
- der Draht (2) einen Überzug (7) aus einem physiologisch verträglichen Stoff aufweist, von dem zumindest die Oberfläche hydrophil und in einer physiologisch verträglichen Flüssigkeit lösbar ist;
- an dem äußeren Ende des Drahtes (2) ein Konnektor (10) befestigt ist, der ein Kopplungselement (23) zum Einführen der Flüssigkeit aufweist;
- der Stoff derart gewählt ist, daß eine möglichst hohe Reibung zwischen Mandrin und Sonde im trockenen Zustand beim Einführen der Flüssigkeit durch Anlösen der hydrophilen Oberfläche herabgesetzt wird, so daß der Mandrin unter möglichst geringer Reibung entfernt werden kann.

2. Sonde nach Anspruch 1,
dadurch **gekennzeichnet,** daß der Überzug (7) aus Gelatine besteht.

3. Sonde nach einem der Ansprüche 1 oder 2,
dadurch **gekennzeichnet,** daß die Gelatine einen Anteil an physiologisch verträglichem Alkohol als Weichmacher aufweist.

4. Sonde nach Anspruch 3,
dadurch **gekennzeichnet,** daß die Gelatine einen Anteil an Formaldehyd oder einem anderen Aldehyd aufweist.

5. Sonde nach einem der vorhergehenden Ansprüche,
dadurch **gekennzeichnet,** daß der Konnektor (10) ein weiteres Kopplungselement (20) aufweist sowie einen beide Kopplungselemente (20, 23) miteinander verbindenden Kanal (24).

6. Sonde nach Anspruch 5,
dadurch **gekennzeichnet,** daß der Konnektor (10) eine Halterung zur Aufnahme einer an einem Drahtende angebrachten Schlinge (4) aufweist.

7. Sonde nach einem der Ansprüche 5 oder 6,
dadurch **gekennzeichnet,** daß der Konnektor (10) aus zwei etwa in einer Axialebene zusammensetzbaren Teilen (12, 13) besteht, in deren Trennebene (11) eine Ausnehmung (19) zur Aufnahme der Drahtschlinge (4) und eines die beiden Kopplungselemente verbindenden Kanals (24) ausgebildet sind.

8. Sonde nach einem der Ansprüche 5 bis 7,
dadurch **gekennzeichnet,** daß das eine Kopplungselement in Form einer Buchse (23) als Paßsitz für den Austrittsstutzen einer Spritze ausgebildet ist.

9. Sonde nach einem der Ansprüche 5 bis 8,
dadurch **gekennzeichnet,** daß das andere Kopplungselement in Form eines Zapfens (20) ausgebildet ist, der in das Mundstück (21) am Ende (26) der Sonde (27) eingreift.

## Claims

1. Probe (6), made from readily flexible materials, comprising a mandrel, in the form of a wire (2), designed to stiffen the probe when introducing the latter into body cavities, wherein the mandrel is removed after the probe has been put into position,
**characterized** in that the wire (2) has a coating (7) of a physiologically compatible substance, of which at least the surface thereof is hydrophilic and soluble in a physiologically compatible liquid,
a connector (10) is attached to the outer end of the wire (2), said connector comprising a coupling member (23) for the introduction of the liquid,
the substance is chosen such that a maximum friction between the mandrel and the probe when they are dry is reduced when introducing the liquid by means of a light dissolution of the hydrophilic surface, such that the mandrel can be removed under minimum friction.

2. Probe according to claim 1,
**characterized** in that the coating (7) consists of gelatin.

3. Probe according to any of claims 1 or 2,
**characterized** in that the gelatin contains a fraction of physiologically compatible alcohol as a softening agent.

4. Probe according to claim 3,
**characterized** in that the gelatin has a fraction of formaldehyde or of another aldehyde.

5. Probe according to any of the preceding claims,
**characterized** in that the connector (10) is provided with another coupling member (20) as well as with a passage (24) interconnecting both coupling members (20, 23).

6. Probe according to claim 5,
**characterized** in that the connector (10) has a holding means for receiving a loop (4) provided at one end of the wire.

7. Probe according to any of claims 5 or 6,
**characterized** in that the connector (10) consists of two parts (12, 13) capable of being put together in a generally axial plane, in the parting plane (11) of which a recess (19) for receiving the wire loop (4) and a passage (24) interconnecting both coupling members are formed.

8. Probe according to any of claims 5 to 7,
**characterized** in that the one coupling member in the form of a sleeve (23) is designed as a snug fit for the outlet nozzle of a syringe.

9. Probe according to any of claims 5 to 8,
**characterized** in that the other coupling member is designed in the form of a spigot (20) which engages with the mouthpiece (21) at the end (26) of the probe (27).

## Revendications

1. Sonde (6) faite de matières très flexibles, comprenant un mandrin formé d'un fil métallique (2) servant à raidir la sonde lorsqu'on l'introduit dans des cavités du corps, le mandrin étant retiré après la mise en place de la sonde,
caractérisée
- en ce que le fil (2) porte un revêtement (7) fait d'une matière physiologiquement compatible dont au moins la surface est hydrophile et soluble dans un liquide physiologiquement compatible ;
- en ce qu'à l'extrémité extérieure du fil (2), est fixé un connecteur (10) qui présente un élément de raccordement (23) pour l'introduction du liquide ;
- en ce que la matière est choisie de manière à abaisser le frottement entre le mandrin et la sonde, qui est très élevé a sec, par dissolution de la surface hydrophile au moment de l'introduction du liquide, de sorte que le mandrin peut ainsi être extrait avec un frottement extrêmement faible.

2. Sonde selon la revendication 1,
caractérisée en ce que le revêtement (7) est composé de gélatine.

3. Sonde selon une des revendications 1 et 2,
caractérisée en ce que la gélatine contient comme plastifiant une proportion d'alcool physiologiquement compatible.

4. Sonde selon la revendication 3,
caractérisée en ce que la gélatine contient une proportion de formaldéhyde ou d'un autre aldéhyde.

5. Sonde selon une des revendications précédentes,
caractérisée en ce que le connecteur (10) présente un autre élément de raccordement (20) ainsi qu'un canal (24) qui relie les deux éléments de raccordement (20, 23) l'un à l'autre.

6. Sonde selon la revendication 5,
caractérisée en ce que le connecteur (10) présente une retenue pour recevoir une boucle (4) placée à une extrémité du fil.

7. Sonde selon une des revendications 5 et 6,
caractérisée en ce que le connecteur (10) est composé de deux parties (12, 13) pouvant être assemblées à peu près dans un plan axial, dans le plan de joint (12) desquelles sont formés un évidement (19) destiné à recevoir la boucle de fil (4), et un canal (24) qui relie les deux éléments de raccordement.

8. Sonde selon une des revendications 5 à 7,
caractérisée en ce qu'un élément de raccordement présentant la forme d'une douille (23) constitue un siège ajusté pour recevoir la tubulure de sortie d'une seringue.

9. Sonde selon une des revendications 5 à 8,
caractérisée en ce que l'autre élément de raccordement forme un téton (20) qui s'engage dans l'embout (21) situé à l'extrémité (26) de la sonde (27).
